# DEMANDE DE BREVET EUROPEEN

(11) **EP 1 041 156 A1**
(43) Date de publication de la demande: **04.10.2000**
(21) Numéro de dépôt: 00400883.5
(22) Date de dépôt: 30.03.2000
(51) Int. Cl.: C12P 19/14, C12P 19/20, C12P 7/18

(54) **Procédé de fabrication d'un hydrolysat d'amidon à haute teneur en dextrose**

(30) Priorité: 02.04.1999 FR 9904177
(71) Demandeur: Roquette Frères, 62136 Lestrem (FR)
(72) Inventeur: Duflot, Pierrick, 62136 Lacouture (FR)
(74) Mandataire: Boulinguiez, Didier

(57) **Abrégé**

L'invention concerne un procédé de fabrication d'un hydrolysat d'amidon à haute teneur en dextrose comprenant les étapes consistant à :
a) effectuer une liquéfaction d'un lait d'amidon à l'aide d'une α-amylase de façon à obtenir un lait d'amidon liquéfié ;
b) effectuer une saccharification du lait d'amidon liquéfié, à l'aide d'une enzyme glucogénique, pour obtenir un hydrolysat saccharifié brut ;
c) effectuer une séparation par nanofiltration sur membranes de l'hydrolysat saccharifié brut de manière à recueillir un perméat de nanofiltration constituant ledit hydrolysat d'amidon à haute teneur en dextrose, et un rétentat de nanofiltration.

## Description

L'invention a pour objet un procédé de fabrication d'un hydrolysat d'amidon à haute teneur en dextrose.

L'invention a également pour objet un procédé de fabrication de sorbitol à partir d'un hydrolysat d'amidon à haute teneur en dextrose obtenu par la mise en oeuvre du procédé conforme à l'invention.

Il est connu de fabriquer des hydrolysats d'amidon pour lesquels la valeur de l'équivalent dextrose (pouvoir réducteur exprimé en glucose sur la matière sèche, ci-après DE) est de 2 à 98 et qui, suivant cette valeur, peuvent renfermer jusqu'à 96 % de dextrose vrai. On obtient ces différentes qualités d'hydrolysats d'amidon par le choix des conditions de l'hydrolyse de l'amidon de départ. La nature de l'hydrolyse, c'est-à-dire le fait que celle-ci soit acide ou enzymatique, intervient également.

Les hydrolysats d'amidon riches en dextrose, bien qu'ayant de nombreux domaines d'application, servent principalement comme matière première pour la fabrication de dextrose cristallisé ou comme substrat pour la fabrication de fructose par isomérisation. Pour ces deux applications, on recherche une conversion la plus élevée possible, c'est-à-dire une teneur la plus élevée possible en dextrose, avec un minimum d'impuretés.

Les procédés de conversion de l'amidon utilisant un acide, conduisent à des hydrolysats d'amidon dont la teneur en dextrose ne dépasse pas 85 à 90 %, ces procédés favorisent en effet les réactions concurrentes de réversion et de déshydratation interne du dextrose.

Les procédés de conversion de l'amidon utilisant à la fois un acide et une enzyme (généralement une glucoamylase) conduisent, eux, à des hydrolysats d'amidon dont la richesse en dextrose n'est jamais supérieure à 93 %. En effet, dans de tels procédés, l'hydrolyse acide de l'amidon produit des saccharides hautement branchés qui résistent à l'action de la glucoamylase.

Les hydrolysats d'amidon obtenus par double conversion enzymatique à l'α-amylase et à l'amyloglucosidase (ou la glucoamylase), titrent généralement de 93 à 95 % de dextrose vrai, et contiennent de 5 à 7 % d'oligosaccharides et de polysaccharides résiduels, dont la majeure partie est constituée de disaccharides (maltose et isomaltose).

Ces hydrolysats sont obtenus de façon classique par une liquéfaction de l'amidon jusqu'à un DE de 12 à 20, suivie d'une saccharification à l'amyloglucosidase, mais dans ces conditions, la teneur en dextrose vrai ne peut dépasser 94 à 95 %.

Afin d'atteindre des teneurs en dextrose vrai supérieures, plusieurs procédés ont été proposés pour, soit améliorer la conversion de l'amidon en limitant la formation des co-produits, soit améliorer l'efficacité de la séparation dextrose/co-produits (oligosaccharides et polysaccharides).

Ainsi un premier procédé consiste à opérer les étapes de liquéfaction et de saccharification à très faibles matières sèches (de l'ordre de 5 à 10 %). Mais même à aussi faible taux de matières sèches, la richesse en dextrose vrai ne dépasse pas 95 à 97 %. En outre un tel procédé n'est pas du tout économiquement rentable du fait de l'énergie nécessaire à l'évaporation de l'eau.

Un autre procédé consiste à opérer la saccharification en présence d'une enzyme hydrolysant les liaisons 1-6 de l'amidon, mais même dans ce cas, la teneur en dextrose n'est au maximum que de 96 à 97 %.

Un autre procédé encore consiste à séparer, de façon connue en soi, le dextrose et les oligosaccharides et polysaccharides par passage de l'hydrolysat sur une colonne d'un tamis moléculaire tel qu'une résine cationique. Dans un tel procédé, l'hydrolysat d'amidon aqueux ayant subi préalablement un prétraitement tel qu'une concentration, une filtration et/ou une décoloration, est adsorbé sur la colonne, les co-produits (les polysaccharides et une partie des oligosaccharides) se retrouvant dans le raffinat exclu du tamis. Le dextrose est ensuite désorbé par élution avec de l'eau, cette dernière étant ensuite partiellement ou complétement éliminée pour former une solution concentrée de dextrose ou du dextrose cristallisé.

Un autre procédé, basé sur le même principe que celui mentionné ci-dessus, consiste à séparer le dextrose et les oligosaccharides et polysaccharides par passage de l'hydrolysat d'amidon sur des membranes à filtration tangentielle. Un tel procédé est décrit dans les documents FR-A-2.762.616 et US-A-5.869.297.

Ce dernier procédé permet effectivement l'obtention d'un hydrolysat d'amidon présentant une teneur élevée en dextrose vrai, supérieure à 98-99 %, mais les rendements obtenus sont malheureusement trop faibles (de l'ordre de 20 à 25 %) pour justifier du point de vue industriel et économique de tels procédés.

Un premier objet de la présente invention est donc de proposer un procédé de fabrication d'un hydrolysat d'amidon à haute teneur en dextrose qui pallie les limites et/ou les inconvénients des procédés connus de l'art antérieur.

Un autre objet de la présente invention est de proposer un procédé de fabrication d'un hydrolysat d'amidon à haute teneur en dextrose, supérieure à 97 % et plus préférentiellement encore supérieure à 99 %.

Un autre objet de la présente invention est de proposer un tel procédé, simple et économiquement performant, permettant d'obtenir, avec des rendements très satisfaisants, des hydrolysats à teneur aussi élevée en dextrose vrai.

A cet effet, l'invention propose un procédé de fabrication d'un hydrolysat d'amidon à haute teneur en dextrose comprenant les étapes consistant à :
(a) effectuer une liquéfaction d'un lait d'amidon à l'aide d'une α-amylase de façon à obtenir un lait d'amidon liquéfié ;
(b) effectuer une saccharification du lait d'amidon liquéfié, à l'aide d'une enzyme glucogénique, pour obtenir un hydrolysat saccharifié brut ;
(c) effectuer une séparation par nanofiltration sur membranes de l'hydrolysat saccharifié brut de manière à recueillir un perméat de nanofiltration constituant ledit hydrolysat d'amidon à haute teneur en dextrose, et un rétentat de nanofiltration.

Après de nombreuses investigations, la Société Demanderesse a constaté que dans un procédé de fabrication d'un hydrolysat d'amidon à haute teneur en dextrose mettant en oeuvre une étape de séparation membranaire, la richesse en dextrose du perméat était meilleure si l'hydrolysat d'amidon saccharifié à séparer était maintenu sous sa forme brute.

Au sens de la présente invention, on entend par hydrolysat d'amidon saccharifié brut, un hydrolysat d'amidon débarrassé de ses matières insolubles et n'ayant subi aucun traitement de purification visant à éliminer les matières solubles (enzymes, protéines, acides aminés, colorants, sels,...) .

Ainsi, contrairement à l'enseignement de l'état de la technique qui prévoit classiquement, en fin de saccharification, une étape d'inhibition de l'enzyme de saccharification (pour éviter la formation de produits de réversion), on cherche donc au contraire dans la présente invention à maintenir une activité enzymatique saccharifiante au sein de l'hydrolysat d'amidon saccharifié.

On cherche également, dans la présente invention, à maintenir la présence de charges au sein de l'hydrolysat d'amidon saccharifié. Dans les procédés conventionnels de l'état de la technique, ces charges sont classiquement éliminées par passage de l'hydrolysat d'amidon saccharifié sur du noir de carbone et sur une résine de déminéralisation. Dans la présente invention, l'hydrolysat n'est pas déminéralisé.

La première étape du procédé conforme à la présente invention consiste donc à liquéfier un lait d'amidon à l'aide d'une α-amylase.

On préfère, avantageusement dans le procédé conforme à l'invention, effectuer une hydrolyse ménagée du lait d'amidon de façon à obtenir un lait d'amidon liquéfié à faible taux de transformation.

Ainsi, dans le procédé conforme à l'invention, on conduit l'étape (a) de liquéfaction de préférence jusqu'à un DE compris entre 2 et 10, et plus particulièrement jusqu'à un DE compris entre 4 et 8.

Le fait d'effectuer la liquéfaction du lait d'amidon à un DE extrêmement faible de 2 à 10 (et de préférence de 4 à 8), en combinaison avec le fait d'inhiber l'enzyme liquéfiante en fin de liquéfaction, favorise l'obtention d'un hydrolysat final présentant les caractéristiques recherchées, c'est-à-dire présentant une teneur en dextrose élevée et une teneur particulière en non dextrose résultant essentiellement de la présence de polysaccharides et non de di- ou oligosaccharides.

De préférence, l'étape de liquéfaction est conduite en deux sous-étapes, la première consistant à chauffer, pendant quelques minutes et à une température comprise entre 105 et 108°C, le lait d'amidon en présence de l'enzyme (type THERMAMYL 120L commercialisée par la société NOVO) et d'un activateur à base de calcium, la seconde consistant à chauffer le lait d'amidon ainsi traité, à une température comprise entre 95 et 100°C pendant une à deux heures.

Une fois l'étape de liquéfaction terminée, dans les conditions de teneur en matières sèches, de pH, de taux d'enzyme et de calcium bien connues de l'homme de métier et après avantageusement inhibition de l'enzyme liquéfiante (en procédant, par exemple, en sortie de liquéfaction à un choc thermique de quelques secondes à une température supérieure ou égale à 130°C), on procède à l'étape (b) de saccharification du lait d'amidon liquéfié.

Lors de cette étape, on soumet le lait d'amidon liquéfié à l'action d'une enzyme glucogénique, notamment choisie dans le groupe constitué de l'amyloglucosidase, la glucoamylase ou toute autre enzyme glucogénique.

Pour éviter les réactions de réversion et la formation notamment de dissaccharides (maltose, isomaltose) par repolymérisation du dextrose, l'étape de saccharification est conduite, dans des conditions et de façon connue en elles-mêmes, pendant au plus 24 heures.

En effet, le substrat préféré des enzymes glucogéniques est de poids moléculaire élevé, et les liaisons α-1,4 de l'amidon sont hydrolysées bien plus rapidement que les liaisons α-1,6. Dès lors, en début de saccharification, les grosses molécules et les liaisons α-1,4 étant prédominantes, la production de dextrose est extrêmement rapide alors que la production des produits de réversion est très lente du fait de la faible concentration en dextrose du milieu réactionnel.

La saccharification se poursuivant, les petites molécules et les liaisons α-1,6 devenant prédominantes, le taux de production du dextrose diminue progressivement alors que la production des produits de réversion (oligosaccharides hautement branchés) s'accélère.

Pour remédier à ce phénomène, il peut être intéressant d'associer à l'enzyme glucogénique une enzyme hydrolysant spécifiquement les liaisons α-1,6 de l'amidon. Cet ajout d'une enzyme débranchante permet d'une part d'accélérer les réactions d'hydrolyse sans simultanément accélérer les réactions de réversion, et d'autre part, de réduire la quantité d'oligosaccharides hautement branchés résistant normalement à l'action de l'enzyme glucogénique. De préférence, l'enzyme débranchante est l'isoamylase ou la pullulanase.

Les quantités et les conditions d'action des différentes enzymes mises en oeuvre dans le procédé conforme à l'invention sont choisies parmi les suivantes :
- α-amylase : 20 à 2,000 KNU (Kilo Novo Units) par kilogramme de substrat sec, température de 80 à 150°C, durée d'action de 2 à 15 minutes.
- amyloglucosidase : 4.000 à 400.000 unités internationales par kilogramme de substrat sec, température de 50°C à 60°C, durée d'action de maximum 24 heures, pH de 4 à 6.
- pullulanase : 150 à 15.000 unités ABM.

Les enzymes utilisées peuvent être d'origine bactérienne ou fongique.

L'hydrolysat ainsi saccharifié est ensuite avantageusement filtré de préférence par microfiltration sur membranes. Les conditions de ce traitement, en particulier sur le plan de la température, sont choisies de manière à maintenir une activité enzymatique saccharifiante au sein de l'hydrolysat d'amidon saccharifié. C'est pourquoi, selon un mode de réalisation préféré de l'invention, on effectue la microfiltration de l'hydrolysat saccharifié brut à une température inférieure ou égale à la température d'inhibition de l'enzyme glucogénique (l'enzyme de saccharification) et, avantageusement, à une température sensiblement équivalente à la température de saccharification. En tout état de cause, la microfiltration est effectuée de préférence à une température inférieure ou égale à 60°C, de préférence à une température de 30°C à 60°C, de préférence encore de 40°C à 50°C. Par exemple, si la température de saccharification est comprise entre 50°C et 60°c, la microfiltration doit s'effectuer de préférence à une température comprise entre 50°C et 60°C.

La membrane de microfiltration mise en oeuvre dans le procédé conforme à l'invention, présente avantageusement une porosité comprise entre 50 nm et 200 nm, ladite porosité étant de préférence de l'ordre de 50 nm. La température opératoire est comprise entre 50°C et 60°C et la pression (transmembranaire) est comprise entre 1 et 2 bars. Une membrane de microfiltration avantageusement mis en oeuvre dans le procédé conforme à l'invention est celle commercialisée par la société SCT (canaux d'un diamètre de 4 mm).

On effectue alors sur cet hydrolysat saccharifié brut, éventuellement microfiltré mais non déminéralisé, une séparation par nanofiltration sur membranes de manière à recueillir un perméat de nanofiltration constituant l'hydrolysat d'amidon à haute teneur en dextrose et un rétentat de nanofiltration.

Contre toute attente, la Société Demanderesse a en effet constaté, à mêmes conditions opératoires, un meilleur enrichissement du perméat en dextrose lorsque l'hydrolysat saccharifié à nanofiltrer était non déminéralisé. Sans vouloir être lié à une quelconque théorie, la Société Demanderesse pense que ce meilleur enrichissement est dû à la formation d'une couche de polarisation plus importante à la surface de la membrane, la formation de cette couche de filtration supplémentaire permettant d'obtenir une richesse en dextrose du perméat plus élevée.

Selon un mode de réalisation préféré, la séparation sur membranes est réalisée sous des conditions de températures inférieures ou égales à 60°C, de préférence comprises entre 30°C et 60°C, de préférence encore comprises entre 40°C et 50°C et de pressions comprises entre 15 et 35 bars, et de préférence comprises entre 20 et 30 bars. Ainsi la membrane de nanofiltration avantageusement mise en oeuvre dans le procédé conforme à l'invention est du type NF40 commercialisée par la société FILMTEC ou du type DESAL 5 DL 3840 commercialisée par la société DESALINATION SYSTEMS.

Selon un mode particulier de réalisation de l'invention, on réalise une saccharification d'au moins une partie du rétentat de nanofiltration de façon à obtenir un rétentat de nanofiltration saccharifié. Cette saccharification secondaire est possible du fait que tout au long du procédé conforme à l'invention, le nécessaire a été fait pour maintenir une activité enzymatique saccharifiante au sein de l'hydrolysat notamment au niveau de l'étape de saccharification en n'inhibant pas l'enzyme glucogénique en fin d'hydrolyse et au niveau de l'étape de microfiltration en travaillant dans des conditions de température similaire à celle de l'étape de saccharification.

On effectue alors sur ce rétentat de nanofiltration saccharifié, qui peut présenter une richesse en dextrose allant jusqu'à 90 %, un tamisage moléculaire de manière à recueillir une fraction enrichie en dextrose et une fraction appauvrie en dextrose.

Cette étape de tamisage moléculaire peut consister, par exemple, en une étape de séparation chromatographique ou en une étape de séparation sur membranes.

L'étape de fractionnement chromatographique est effectué de manière connue en soi, de façon discontinue ou continue (lit mobile simulé), sur des adsorbants du type résines cationiques, ou sur des zéolithes fortement acides, chargées préférentiellement à l'aide d'ions alcalins ou alcalino-terreux tels que le calcium ou le magnésium mais plus préférentiellement à l'aide d'ions sodium.

Selon un mode de réalisation préféré, le fractionnement chromatographique est réalisé en employant le procédé et l'appareillage décrits dans le brevet américain US-A-4 422 881 dont la société demanderesse est titulaire. Quel que soit le procédé chromatographique retenu, on a recours de préférence en ce qui concerne l'adsorbant, à une résine cationique forte employée sous forme sodium ou potassium et réticulée avec environ 4 à 10 % de divinylbenzène. Les résines sont avantageusement de granulométrie homogène et comprise entre 100 et 800 micromètres.

Le choix des paramètres de fractionnement chromatographique, parmi lesquels on note plus particulièrement le débit d'élution, le débit d'alimentation en hydrolysat de départ, le débit d'extraction de la fraction contenant l'hydrolysat d'amidon à teneur élevée en dextrose, le débit de la fraction contenant les impuretés de poids moléculaire élevé et la composition des zones de désorption, d'adsorption et d'enrichissement est effectué de telle manière que l'on récupère, avec un rendement le meilleur possible, la première fraction enrichie en dextrose ayant une teneur en dextrose supérieure à 99 %.

Pour parvenir à ce résultat, ces paramètres sont choisis comme suit quand le fractionnement chromatographique est réalisé en utilisant les procédé et appareillage décrits dans le brevet US 4 422 881 et quand l'adsorbant utilisé est une résine cationique de faible granulométrie réticulée à 8 % de divinylbenzène et qu'elle est utilisée sous forme sodium :
- débit d'élution de 70 à 700 l/h/m³ d'adsorbant,
- débit d'alimentation en hydrolysat de départ de 10 à 100 l/h/m³ d'adsorbant,
- débit d'extraction de la fraction enrichie en dextrose de 80 à 800 l/h/m³
- débit d'extraction de la fraction enrichie en dextrose de 80 à 800 l/h/m³ d'adsorbant,
- débit de la fraction enrichie en oligo- et polysaccharides de 20 à 200 l/h/m³ d'adsorbant.

En lieu et place de l'étape de séparation chromatographique, il est possible, dans le procédé conforme à l'invention, de mettre en oeuvre une étape de séparation par nanofiltration sur membranes, du type de celle décrite ci-dessus.

La fraction enrichie en dextrose obtenue en sortie de l'étape de chromatographie ou de nanofiltration peut alors être mélangée avec l'hydrolysat d'amidon à haute teneur en dextrose précédemment obtenu.

Selon un mode de réalisation préféré de l'invention, le procédé qui vient d'être décrit est continu.

Cet hydrolysat (ainsi que la fraction appauvrie en dextrose) obtenu conformément au procédé de l'invention peut alors être facilement hydrogéné catalytiquement.

L'hydrogénation d'un tel hydrolysat s'effectue conformément aux règles de l'art qui conduisent par exemple à la production de sorbitol à partir du glucose.

On peut utiliser pour cette étape aussi bien des catalyseurs à base de ruthénium que des catalyseurs au nickel de Raney. On préfère cependant utiliser des catalyseurs au nickel de Raney qui sont moins onéreux.

Dans la pratique, on utilise de 1 à 10 % en poids de catalyseur par rapport à la matière sèche de l'hydrolysat soumis à l'hydrogénation. L'hydrogénation s'effectue de préférence sur un hydrolysat dont la matière sèche est comprise entre 15 et 50 %, dans la pratique voisine de 30 à 45 %, sous une pression d'hydrogène comprise entre 20 et 200 bars. Elle peut être effectuée de manière continue ou discontinue.

Lorsque l'on opère de manière discontinue, la pression d'hydrogène utilisée est généralement comprise entre 30 et 60 bars et la température à laquelle se déroule l'hydrogénation est comprise entre 100 et 150°C. On veille aussi à maintenir le pH du milieu d'hydrogénation par l'addition de soude ou de carbonate de soude par exemple, mais sans dépasser un pH de 9,0. Cette manière de faire permet d'éviter l'apparition de produits de crackage ou d'isomérisation.

On arrête la réaction lorsque la teneur du milieu réactionnel en sucres réducteurs est devenue inférieure à 1 %, de préférence encore inférieure à 0,5 % et plus particulièrement inférieure à 0,1 %.

Après refroidissement du milieu réactionnel, on élimine le catalyseur par filtration et on déminéralise le sorbitol ainsi obtenu sur des résines cationiques et anioniques. A ce stade, les sirops contiennent au moins 98 % de sorbitol et il est aisé de purifier celui-ci par cristallisation après concentration et refroidissement des solutions.

D'autres caractéristiques et avantages de l'invention apparaîtront clairement à la lecture des exemples qui suivent. Ils ne sont toutefois donnés ici qu'à titre illustratif et non limitatif.

### EXEMPLE 1

Un lait d'amidon est liquéfié de manière classique à l'aide de 0,5 pour mille de THERMAMYL 120L (α-amylase commercialisée par la société NOVO) jusqu'à un DE de 6,5.

On chauffe ensuite le milieu réactionnel pendant quelques secondes à 140°C de manière à inhiber l'α-amylase.

On effectue alors, de manière connue en soi, la saccharification de l'hydrolysat à 35 % de matières sèches en présence de 0,8 pour mille d'amyloglucosidase G990 commercialisée par la société ABM (température : 60°C, pH = 4,5).

Après 24 heures de saccharification, on obtient un hydrolysat ayant le spectre glucidique suivant :

| | |
|---|---|
| glucose | 93 % |
| DP2 | 2,5 % |
| DP3 | 0,5 % |
| DP supérieurs | 4 % |

étant entendu que l'abbréviation "DP" signifie degré de polymérisation.

L'activité enzymatique mesurée est de 3 U/l.

L'hydrolysat ainsi saccharifié est ensuite filtré par microfiltration sur membranes.

Les conditions opératoires sont les suivantes :
- Membrane SCT : 50nm
- Température : 60°C
- Pression : 2 bars

L'activité enzymatique mesurée est de 2,5 U/l

L'hydrolysat ainsi microfiltré est séparé en deux pour constituer un hydrolysat A et un hydrolysat B.

Conformément à la présente invention, l'hydrolysat A n'est pas déminéralisé. L'hydrolysat B est quant à lui déminéralisé par passage sur noir de carbone et résine.

Chacun des hydrolysats A et B est soumis à une nanofiltration sous les conditions opératoires suivantes :
- Membrane DESAL 5 DL
- Température : 45°C
- Pression : 25 bars

Les caractéristiques des perméats et rétentats de nanofiltration A et B des hydrolysats A et B sont les suivantes :

| | Dextrose / pureté | Activité enzymatique |
|---|---|---|
| Perméat A | 99,7 % | 0 U/l |
| Rétentat A | 80 % | 7 U/l |
| Perméat B | 98,5 % | 0 U/l |
| Rétentat B | 80 % | 0 U/l |

### EXEMPLE 2

Le rétentat A conforme à l'invention obtenu dans l'exemple 1 est ajusté à une matière sèche de 30 % et ramené à 60°C et pH 4,5. Sous l'action de l'activité enzymatique concentré de l'amyloglucosidase, la richesse en dextrose de ce rétentat passe de 80 % à 90 % en 24 heures de saccharification.

### EXEMPLE 3

Le perméat A de l'exemple 1, purifié puis concentré à une matière sèche de 45 %, est soumis à une hydrogénation catalytique en présence de 5 % en poids par rapport à la matière sèche de nickel de Raney.

Les conditions opératoires sont les suivantes :

| | |
|---|---|
| température | 140°C |
| pression | 60 bars |
| durée | 2 heures |

On arrête l'hydrogénation lorsque la teneur du milieu réactionnel en sucres réducteurs est inférieure à 600 ppm.

Après refroidissement du milieu réactionnel, on élimine le catalyseur par filtration, puis on déminéralise le sirop obtenu et enfin on le concentre à 70 % de matières sèches.

La composition sur sec du sirop ainsi obtenu est la suivante :

| | |
|---|---|
| sorbitol | 98,6 % |
| mannitol | 0,4 % |
| iditol et produits de crackage | 0,2 % |

## Revendications

1. Procédé de fabrication d'un hydrolysat d'amidon à haute teneur en dextrose comprenant les étapes consistant à :
a) effectuer une liquéfaction d'un lait d'amidon à l'aide d'une α-amylase de façon à obtenir un lait d'amidon liquéfié ;
b) effectuer une saccharification du lait d'amidon liquéfié, à l'aide d'une enzyme glucogénique, pour obtenir un hydrolysat saccharifié brut ;
c) effectuer une séparation par nanofiltration sur membranes de l'hydrolysat saccharifié brut de manière à recueillir un perméat de nanofiltration constituant ledit hydrolysat d'amidon à haute teneur en dextrose, et un rétentat de nanofiltration.

2. Procédé selon la revendication 1, caractérisé par le fait que l'on effectue la nanofiltration à une température inférieure ou égale à la température d'inhibition de l'enzyme glucogénique.

3. Procédé selon la revendication 1 ou 2, caractérisé par le fait que l'on effectue l'étape (b) de sacccharification pendant au maximum 24 heures.

4. Procédé selon l'une quelconque des revendications 1 ou 3, caractérisé par le fait que l'on effectue une microfiltration de l'hydrolysat saccharifié brut.

5. Procédé selon la revendication 4, caractérisé par le fait que l'on effectue la microfiltration à une température inférieure ou égale à la température d'inhibition de l'enzyme glucogénique.

6. Procédé selon la revendication 5, caractérisé par le fait que l'on effectue la microfiltration à une température sensiblement équivalente à la température de saccharification.

7. Procédé selon l'une quelconque des revendications 1 à 6, caractérisé par le fait que l'on effectue une saccharification d'au moins une partie du rétentat de nanofiltration, de façon à obtenir un rétentat de nanofiltration saccharifié.

8. Procédé selon la revendication 7, caractérisé par le fait que l'on effectue un tamisage moléculaire dudit rétentat de nanofiltration saccharifié de manière à recueillir une fraction enrichie en dextrose et une fraction appauvrie en dextrose.

9. Procédé selon la revendication 7 ou 8, caractérisé par le fait que l'on mélange ladite fraction enrichie en dextrose avec ledit hydrolysat d'amidon à haute teneur en dextrose.

10. Procédé de fabrication de sorbitol par hydrogénation d'un hydrolysat d'amidon à haute teneur en dextrose, caractérisé par le fait que ledit hydrolysat est obtenu par la mise en oeuvre du procédé conforme à l'une quelconque des revendications 1 à 9.
